Europäisches Patentamt

European Patent Office

Office européen des brevets

(11) Publication number: **0 034 872**
**B1**

# EUROPEAN PATENT SPECIFICATION

(45) Date of publication of patent specification: **27.11.85**

(21) Application number: **81200219.4**

(22) Date of filing: **24.02.81**

(51) Int. Cl.⁴: **C 02 F 11/18, C 02 F 11/12, C 12 N 1/02, A 23 K 1/00**

(54) **Process of and installation for dehydrating protein-comprising sludge.**

(30) Priority: **25.02.80 NL 8001128**

(43) Date of publication of application:
**02.09.81 Bulletin 81/35**

(45) Publication of the grant of the patent:
**27.11.85 Bulletin 85/48**

(84) Designated Contracting States:
**AT BE CH DE FR GB IT LI LU NL SE**

(56) References cited:
**CH-A- 227 358**
**CH-A- 491 824**
**GB-A- 196 239**
**GB-A- 420 720**
**GB-A-1 192 848**
**US-A-3 936 375**

(73) Proprietor: **Ottens,Erroll Paul Karel, Dr.Ir.**
**33 't Corso**
**NL-8408 HW Lippenhuizen (NL)**

(72) Inventor: **Ottens,Erroll Paul Karel, Dr.Ir.**
**33 't Corso**
**NL-8408 HW Lippenhuizen (NL)**

(74) Representative: **van der Veken, Johannes Adriaan et al**
**EXTERPATENT Willem Witsenplein 4**
**NL-2596 BK 's-Gravenhage (NL)**

The file contains technical information submitted after the application was filed and not included in this specification

Courier Press, Leamington Spa, England.

## Description

The invention relates to a process of dewatering pre-coagulated protein-comprising aqueous sludge obtained in installations adapted to purify waste water, wherein a heated gaseous fluid having a temperature of over 80°C is passed into the aqueous sludge whereupon the agglomerated sludge is separated.

Such a method is known from US—A—3.936.375 wherein steam is directly passed into the sludge which is heated to a temperature of 95°C.

This method presents the disadvantage that prior to the steam treatment calcium chloride must be added and the pH has to be adjusted to 6,5 to 9,5.

Furthermore it has been found that in the presence of fats, being nearly always present in such aqueous sludges from slaughter houses the proteins are difficult to remove by agglomeration.

GB—A—1.192.848 discloses a process of dewatering sludge by heating waste liquid together with a coagulant by heating to a temperature comprised between 50 and 100°C.

This method does not provide a complete agglomeration of proteins.

GB—A—420.720 discloses a plant for treatment of sewage wherein the sewage is heated and after heating the heated sewage is charged into receptacles. One receptacle is emptied when another receptacle is filled.

It is now the aim of the invention to provide a simple method for agglomerating the proteins which does not present the aforementioned disadvantages.

This aim is attained in that the during the introduction of heated gaseous fluid the aqueous sludge is subjected to rotatory shear forces.

In a preferred embodiment the rotatory shear forces are created by injecting heated gaseous fluid through and tangentially to the wall of a cylindrical zone containing said aqueous sludge. The mixture to be treated will then rotate along the steam injection points, which rotation will create shear forces involving the desired agglomeration of the protein particles.

If the initial sludge comprises fat, the agglomeration effect is generally improved, although the size of the agglomerates will not increase substantially. Free fatty acids and dissolved fats are mainly responsible for the increased binding effect between the protein particles.

If the sludge comprises a fat, the mass should be cooled to a temperature ranging from 20 to 25°, prior to filtration. Due to this cooling dissolved fats and fatty acids can be bonded to the previously obtained agglomerates. If a direct filtration is carried out, which means at higher temperatures, dissolved fats and fatty acids will be entrained with the filtrate, which is undesirable.

Agglomeration is preferably carried out by injecting a low pressure steam 101,0 to 107,9 kPa (1,03 to 1,10 kg/cm$^2$) into the sludge during a short period of about 20 seconds, in such a manner that a rotating movement of the sludge along the steam injection points or openings is obtained.

The agglomeration of protein flakes or agglomerates as already present in the sludge, can be explained as follows.

Passing steam into the sludge which causes e.g. a temperature rise, will involve a change of the surface structure of the protein flakes or agglomerates as present so that the flakes or agglomerates tend to stick to one another and form larger flakes or agglomerates under the influence of the rotatory shear forces, which larger flakes are more easily dewatered. The presence of a stirring means or other additional means so as to effect said rotation, is not essential, the more so as such additional mechanical means will decrease the size of the formed agglomerates.

The agglomeration may simply be effected by an injection velocity of steam ranging from 60 to 80 m/sec. at the injection points or openings.

Said agglomeration may both be effected in a discontinuous or continuous manner.

In order to carry out a discontinuous agglomeration, a cylindrical vessel may be used, which is initially filled with non-agglomerated sludge. Subsequently, steam is injected for a period of 20 seconds, after which period of time the injection of steam is stopped, whereupon the agglomeration vessel can be emptied and said treatment is subsequently repeated.

In a continuous agglomeration process fresh sludge and gaseous fluid are simultaneously passed into the cylindrical vessel, for example through the bottom of said vessel. The agglomerated sludge can then leave the agglomeration vessel through an overflow. The average residence time of the sludge in the vessel or reactor should amount to approximately 20 seconds.

Immediately after agglomeration of protein-containing sludge the mass may be filtered upon a conventional sieve filter belt. The filtrate is clear and has a relatively high temperature of approximately 90°C.

The process in accordance with the invention may be optimized as regards energy conservation by performing the dehydration in three subsequent steps if the dry substance contents of the final sludge is comprised between 35 and 50%.

Should said sludge be dewatered to a dry substance contents ranging from approximately 80 to 95%, a fourth subsequent step can be performed. The aforementioned three subsequent treatments of sludge being practically devoid of fat, comprise the following steps:

1. a preheating of the sludge up to a temperature of about 70°C;

2. agglomeration of the sludge by means of a direct injection of steam and a simultaneous rotation of the mass along the steam injection points or openings, thus providing shear forces;

3. filtration of the sludge agglomerates, whereby the obtained filtrate having a high temperature, is used for preheating the sludge in the first step.

If the sludge has to be dewatered to a higher extent, the fourth step is:

4. drying the sludge by means of blowing hot air into the sludge or providing a good contact of the sludge as filtered off, and air.

In the case that a separate protein-comprising sludge and a separate fat-comprising sludge can be recovered during a chemicophysical purification, preferably only the protein-comprising sludge should be subjected to the aforementioned four steps, whilst the fat which can be thickened by means of exhaust drying air from the dryer (fourth step) is added after having dried the protein-comprising sludge.

If a combined protein and fat-comprising sludge is obtained, during a chemico-physical purification, the agglomerated sludge has first to be cooled down to a temperature comprised between e.g. 20 and 25°C, so as to solidify the fat which has become liquid, as otherwise liquid fat would for the greater part leave the installation with the filtrate of the sludge agglomerate filtration.

The invention also comprises an installation for dewatering pre-coagulated protein containing aqueous sludge from an installation adapted to purify waste water according to a process according to the invention, characterized in that the installation comprises a vessel provided with an inlet for heated gaseous fluid opening into said vessel and an inlet for pre-coagulated protein-comprising aqueous waste sludge, and the vessel is provided with means for subjecting the aqueous sludge to rotatory shear forces during introduction of heated gaseous fluid and said installation comprises separating means for the separation of liquids from agglomerated proteins.

It should be noted that CH—A—227.358 discloses a device for softening boiler water comprising a vessel provided with a tangential inlet pipe arrangement at the base of the vessel for supplying water and steam for producing a stirring action. However, this device is not provided with separating means for the separation of liquids from agglomerated proteins.

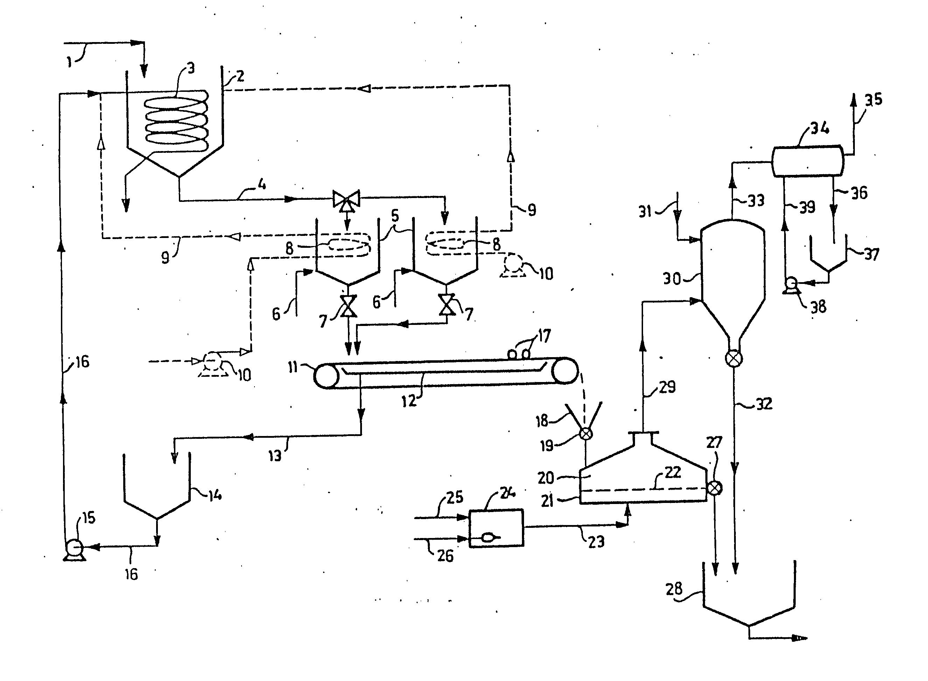

The invention will now be illustrated with respect to the drawing which shows an installation for processing and dewatering protein-containing sludge, the agglomeration of the protein beng performed discontinuously.

Pre-coagulated protein-containing sludge from a physicochemical plant for the purification of waste water of a slaughter house is passed into the container 2 through a line 1, starting from a sludge source (not shown). Said container 2 comprises a heat exchanger 3, for heating the sludge up to a temperature comprised between 60 to 70°C.

The container 2 may advantageously be provided with a double wall, for the passage of a heated aqueous fluid, which may consist e.g. of filtration water, obtained in a step to be described later on.

Depending on the dry substance concentration in the sludge, it may be recommended to provide the container 2 with a stirring means for stirring the sludge mixture.

From container 2 the sludge is conveyed to an agglomeration vessel 5 through line 4, forming the inlet for pre-coagulated sludge from container 2.

The drawing shows two agglomeration vessels, thus illustrating that the process according to the invention is carried out discontinuously.

One vessel 5a being filled and steam being injected, so that agglomeration of the proteins is initiated, the other vessel or reactor 5b is emptied.

Depending on the capacity of the installation, either a plurality of semi-continuously operating agglomeration vessels may be used, or one continuously operating agglomeration vessel from which the agglomerated sludge is conveyed to a filter 11, via an overflow.

Steam is tangentially injected into the bottom part of the agglomeration vessel 5 through an inlet 6, the steam velocity at the injection points ranging from 60 to 80 m/sec.

This tangential injection creates shear forces in the sludge material. The applied steam is a low pressure steam having a pressure comprised between 101,0 to 107,9 kPa (1,03 and 1,1 kg/cm$^2$).

During a short period of time of 20 seconds, the proteins are sufficiently heated, thus causing these proteins to agglomerate and to easily precipitate.

The introduction of steam provides the advantage that a pasteurization occurs, which prevents any bacteriological problems during further processing.

The dimensions of the vessel 5 are such that the height of the vessel is twice as large as the diameter.

In a discontinuous embodiment the upper side of each agglomeration vessel 5 is sealed off, but said vessel may also be provided with a deaerating means.

After having terminated the injection of steam, to wit after a period of 20 seconds, the vessel is emptied by opening valve 7.

Said vessel 5 may comprise a cooling spiral or a double wall, thus permitting cooling of the agglomerated mass. This is particularly important if the starting material contains considerable quantities of fat.

The discharged cooling water can be pumped by pump 10 through circuit 9 towards the heat exchanger 3 of the sludge container 2.

As described hereinbefore, vessel 5 is emptied by means of a valve 7, said emptying operation causing the material to flow across filter 11. The filtrate obtained thereby is recovered in a recipient 12 and is conveyed to a storage container 14, via a line 13 from which storage container the filtrate can be conveyed through line 13, towards the heat exchanger 3 of the sludge container 2, by means of a pump 15.

The heat exchanger or the double wall of container 2 are connected with the line 16.

The filter 12 may be provided with a plurality of pressure rollers 17 so as to press a considerable quantity of still adhering liquid out of the filtered sludge.

The filter cake is fed to a dryer 20 through a supply hopper 18 and a dual valve mechanism 19.

Said dryer 20 consists of a housing 21, comprising a metal sheet 22 being provided with perforations in the form of holes or slots.

Hot air is supplied through air channel 23 below metal sheet 22, which hot air flows through the holes or slots of said metal sheet 22, thus causing a good contact between the sludge to be dried and the hot air.

The air is preferably heated by means of an air heater 24, being fed with fuel through a line 26, and with air through a line 25.

The dryer 20 is preferably a shaking or vibration-bed-dryer so that a controlled residence time division of the material to be dried can be obtained. Via a double valve mechanism, the dried material can be stored in a bunker and forms a suitable fodder material.

The exhaust air from dryer 20 can be discharged through an air line 29. If the material comprises considerable quantities of fat, said fat can be thickened in a thickening device 30, which is supplied with said fat through a line 31, whilst the heated gases required for said thickening operation then consist of drying air escaped from dryer 20 after drying the agglomerated proteins, which air is supplied through line 29, said air has a temperature of approximately 70°C.

The thickened fat is supplied to the previously dried protein-containing sludge in bunker 28 via line 32. The exhaust air from the fat thickener 30 is discharged through line 33. Should the latter exhaust air give rise to possible environmental odour problems, said air can still be washed by injecting a liquid in the form of e.g. a diluted sodium hypochlorite solution into the air washer 34. The liquid is passed into the air washer 34 through line 39 and is discharged through line 36. The air is discharged through line 35. The liquid is recycled by means of a pump 38, through a recovering container 37.

The airwasher may have the form of a hollow cylinder, comprising a plurality of spray bodies directed towards the direction of air current.

By choosing the air velocity, sufficiently small at the point of discharge of the air, drops can be caught in a very efficient manner.

In order to obtain pre-coagulated protein-containing sludge, additives, such as ferric chloride or polyelectrolytes are advantageously added to a protein containing waste liquid, thus causing said proteins to flocculate or to pre-coagulate. Said flocculated or pre-coagulated protein forming an emulsion will not precipitate without using the method in accordance with the invention.

Hitherto said flocculated proteins were subjected to flotation whereupon the obtained protein floating layer was removed. This method presents the disadvantage of being very expensive.

## Claims

1. Process of dewatering pre-coagulated protein-comprising aqueous sludge obtained in installations adapted to purify waste water, wherein a heated gaseous fluid having a temperature of over 80°C is passed into the aqueous sludge whereupon the agglomerated sludge is separated, characterized in that the during the introduction of heated gaseous fluid the aqueous sludge is subjected to rotatory shear forces.

2. Process according to claim 1, characterized in that the rotatory shear forces are created by injecting heated gaseous fluid through and tangentially to the wall of a cylindrical zone containing said aqueous sludge.

3. Process according to claim 1 or 2, characterized in that the heated gaseous fluid is steam having a temperature of over 100°C and having preferably a pressure ranging from 101,0 to 107,9 kPa (1,03 to 1,10 kg/cm$^2$).

4. A process according to claims 1 to 3, characterized in that the agglomerated protein-containing sludge is filtered off and the filtrate obtained by filtering the agglomerated sludge, is used for pre-heating sludge to be treated.

5. Process according to any one of claims 1 to 4, characterized in that if the aqueous sludge contains fats the agglomerated aqueous sludge is cooled to a temperature below the solidification point of said fats, prior to the filtration.

6. Installation for dewatering pre-coagulated protein-containing aqueous sludge from an installation adapted to purify waste water according to a process according to claims 1 to 5, characterized in that the installation comprises a vessel (5) provided with an inlet (6) for heated gaseous fluid opening into said vessel and an inlet (4) for pre-coagulated protein-comprising aqueous waste sludge, and the vessel (5) is provided with means (6) for subjecting the aqueous sludge to rotatory shear forces during introduction of heated gaseous fluid and said installation comprises separating means (11) for the separation of liquids from agglomerated proteins.

7. Installation according to claim 6, characterized in that the inlet (6) for heated gaseous fluid debouches tangentially into the wall of the cylindrical vessel (5).

8. Installation according to claims 6 or 7, characterized in that the installation comprises cooling means (8) for cooling the agglomerated aqueous sludge composition before feeding said separating means (11).

## Patentansprüche

1. Verfahren zum Entwässern von vorkoaguliertem, proteinhaltigem, wäßrigem Schlamm, wie er in zur Reinigung von Abwasser geeigneten Vorrichtungen anfällt, wobei ein erhitztes, gasförmiges Fluid mit einer Temperatur von über 80°C in den wäßrigen Schlamm eingeleitet wird, woraufhin der agglomerierte Schlamm abgetrennt wird, dadurch gekennzeichnet, daß der wäßrige Schlamm während dem Einleiten des erhitzten, gasförmigen Fluids Rotationsscherkräften ausgesetzt wird.

2. Verfahren nach Anspruch 1, dadurch gekennzeichnet, daß die Rotationsscherkräfte durch Einführen des erhitzten, gasförmigen Fluids durch und tangential zu der Wand einer zylindrischen Zone, die den wäßrigen Schlamm enthält, erzeugt werden.

3. Verfahren nach Anspruch 1 oder 2, dadurch gekennzeichnet, daß das erhitzte, gasförmige Fluid Dampf ist, der eine Temperatur von über 100°C und vorzugsweise einen Druck im Bereich von 101,0 bis 107,9 kPa (1,03 bis 1,10 kg/cm$^2$) aufweist.

4. Verfahren nach den Ansprüchen 1 bis 3, dadurch gekennzeichnet, daß der agglomerierte, proteinhaltige Schlamm abfiltriert wird und das beim Filtrieren des agglomerierten Schlammes erhaltene Filtrat zum Vorerhitzen von zu behandelndem Schlamm verwendet wird.

5. Verfahren nach einem der Ansprüche 1 bis 4, dadurch gekennzeichnet, daß, wenn der wäßrige Schlamm Fette enthält, der agglomerierte, wäßrige Schlamm vor dem Filtrieren auf eine Temperatur unterhalb des Verfestigungspunkts der Fette gekühlt wird.

6. Vorrichtung zum Entwässern von vorkoaguliertem, proteinhaltigem, wäßrigem Schlamm aus einer zur Reinigung von Abwasser geeigneten Vorrichtung nach einem Verfahren gemäß den Ansprüchen 1 bis 5, dadurch gekennzeichnet, daß die Vorrichtung einen Behälter (5), der mit einem in den Behälter einmündenden Einlaß (6) für ein erhitztes, gasförmiges Fluid und einem Einlaß (4) für vorkoagulierten, proteinhaltigen, wäßrigen Abfallschlamm versehen ist, umfaßt, und daß der Behälter (5) mit Mitteln (6) versehen ist, um den wäßrigen Schlamm während der Einführung von erhitztem, gasförmigem Fluid Rotationsscherkräften auszusetzen, und daß die Vorrichtung Abtrenneinrichtungen (11) zur Abtrennung von Flüssigkeiten aus agglomerierten Proteinen umfaßt.

7. Vorrichtung nach Anspruch 6, dadurch gekennzeichnet, daß der Einlaß (6) für das erhitzte, gasförmige Fluid tangential in die Wand des zylindrischen Behälters (5) einmündet.

8. Vorrichtung nach Anspruch 6 oder 7, dadurch gekennzeichnet, daß die Vorrichtung Kühleinrichtungen (8) zum Kühlen der agglomerierten wäßrigen Schlammzusammensetzung vor dem Zuführen zu den Abtrenneinrichtungen (11) umfaßt.

**Revendications**

1. Un procédé de déshydratation d'une boue ou suspension précoagulée aqueuse contenant des protéines provenant d'installations de purification d'eaux usées, dans lequel on fait passer un fluide gazeux chauffé ayant une température supérieure à 80°C dans la boue aqueuse, ce après quoi on sépare la boue agglomérée, caractérisé en ce que, pendant l'introduction du fluide gazeux chauffé, la boue aqueuse est soumise à des forces de cisaillement rotatives.

2. Un procédé selon la revendication 1, caractérisé en ce que les forces de cisaillement rotatives sont créées par injection d'un fluide gazeux chaud à travers et tangentiellement à la paroi d'une zone cylindrique contenant cette boue aqueuse.

3. Un procédé selon la revendication 1 ou 2, caractérisé en ce que le fluide gazeux chauffé est une vapeur ayant une température supérieure à 100°C et ayant de préférence une pression allant de 101,0 à 107,9 kPa (1,03 à 1,10 kg/cm$^2$).

4. Un procédé selon les revendications 1 à 3, caractérisé en ce que la boue contenant des protéines agglomérées est séparée par filtration et le filtrat obtenu en filtrant la boue agglomérée est utilisé pour préchauffer la boue à traiter.

5. Un procédé selon l'une quelconque des revendications 1 à 4, caractérisé en ce que, si la boue aqueuse contient des matières grasses, la boue aqueuse agglomérée est refroidie, avant filtration, à une température inférieure aux points de solidification de ces matières grasses.

6. Une installation de déshydratation d'une boue aqueuse contenant des protéines précoagulées provenant d'une installation de purification d'eaux usées, à l'aide du procédé selon les revendications 1 à 5, caractérisée en ce que l'installation comprend un récipient (5) pourvu d'une entrée (6) du fluide gazeux chauffé et une conduite (4) pour la boue ou suspension aqueuse précoagulée contenant des protéines formant l'effluent et que le récipient (5) est pourvu de moyens (6) pour soumettre la boue aqueuse à des forces de cisaillement rotatives pendant l'introduction du fluide gazeux chauffé, cette installation comprenant des moyens de séparation (11) pour séparer les liquides et les protéines agglomérées.

7. Une installation selon la revendication 6, caractérisée en ce que l'entrée (6) du fluide gazeux chauffé débouche tangentiellement dans la paroi du récipient cylindrique (5).

8. Une installation selon la revendication 6 ou 7, caractérisée en ce que l'installation comprend des moyens de refroidissement (8) pour refroidir la composition de boue aqueuse agglomérée avant son introduction dans ces moyens de séparation (11).

0 034 872